# EUROPÄISCHE PATENTSCHRIFT

(11) **EP 1 154 778 B1**
(45) Veröffentlichungstag und Bekanntmachung des Hinweises auf die Patenterteilung: **12.05.2004**
(21) Anmeldenummer: 00916855.0
(22) Anmeldetag: 22.02.2000
(51) Int. Cl.: A61K 31/683, A61K 31/665, A61P 17/06

(54) **PHARMAZEUTISCHE ZUBEREITUNG ENTHALTEND DITHRANOL**
PHARMACEUTICAL PREPARATION CONTAINING DITHRANOL
PREPARATION PHARMACEUTIQUE CONTENANT DU DITHRANOL

(30) Priorität: 26.02.1999 DE 19908487
(43) Veröffentlichungstag der Anmeldung: 21.11.2001
(73) Patentinhaber: Esparma GmbH, 39171 Osterweddingen (DE)
(72) Erfinder: Schneider, Jürgen, D-06179 Angersdorf (DE); Wohlrab, Wolfgang, D-06110 Halle (DE); Neubert, Reinhard, D-06120 Halle (DE); Huschka, Christoph, D-06110 Halle (DE); Koegst, Dieter, 39171 Osterweddingen (DE); Fries, Gerhard, 39171 Osterweddingen (DE)
(74) Vertreter: Pfenning, Meinig & Partner
(86) Internationale Anmeldenummer: PCT/EP2000/001421
(87) Internationale Veröffentlichungsnummer: WO 2000/050018

(56) Entgegenhaltungen:
- DE-A- 2 302 125
- DE-A- 4 231 636
- US-A- 3 881 000
- US-A- 5 358 716

## Beschreibung

Die Erfindung betrifft eine pharmazeutische Zubereitung in einer zur äußeren Anwendung geeigneten Aufmachungsform, die Phosphorsäureester des Dithranols enthält und deren Verwendung zur reizarmen Therapie der Psoriasis.

Dithranol ist einer der bewährtesten Arzneistoffe zur Therapie der Psoriasis in der Dermatologie. Die Psoriasis gehört nach wie vor zu Dermatosen mit bisher ungeklärter Ethologie. Zur klassischen externen Behandlung werden deshalb verschiedene Zubereitungen unter anderem mit Dithranol, Salizylsäure oder Harnstoff herangezogen. Ein wesentlicher Nachteil bisheriger pharmazeutischer Zubereitungen mit Dithranol war die Verwendung von lipophilen Grundlagen verursacht durch die hohe Lipophilie des Wirkstoffes aus denen er sehr schlecht in erkrankte, weniger lipophile Hautpartien penetrieren kann. Gewöhnlich werden für Dithranol lipophile Salbengrundlagen als Vehikel eingesetzt. Der Wirkstoff ist wasserunlöslich und kann nur unter Zusatz größerer Mengen von Tensiden bei geringer Wirkstoffstabilität partiell in wäßrige Formulierungen eingebracht werden. Der guten therapeutischen Wirksamkeit von Dithranol steht jedoch seine stark färbende Eigenschaft entgegen, die zu geringer Akzeptanz bei den Patienten und erheblichen Reinigungsproblemen bei insbesondere klinischer Behandlung führen. Bei der Untersuchung der Wirkungsweise von Dithranol konzentrieren sich die Arbeiten auf die Aufklärung der Radikalbildungsprozesse in vivo. Diese Prozesse sind sehr komplexer Natur, legen jedoch nahe, daß insbesondere die Oxidation des Anthrons zum Anthrachinon über radikalische Zwischenstufen abläuft und somit auch als Beleg für die Wirksamkeit von phototherapeutischen Behandlungsmethoden gelten kann.

Ausgehend hiervon, d.h. von der Tatsache, daß Dithranol nach wie vor ein sehr effektiver Arzneistoff zur Behandlung der Psoriasis ist (z.B. W.C. Marsch, DAZ, 1995 135(38), 3498-3499), gibt es zahlreiche Bestrebungen zu seiner Derivatisierung (K.K. Mustakallio, Acta Derm. Venerol. (Stockh.), 1992, 172 Suppl., 7-9). Derivate von Dithranol sind in der DE 4 231 636 A1, US 5,352,716, DE-OS 2 302 155 sowie der US 3,881,000 beschrieben. Der überwiegende Teil aller Untersuchungen ist dabei auf Derivate konzentriert, die vorzugsweise in 10-Position neue Substituenten aufweisen. Es konnte gezeigt werden, daß die antipsoriatische Wirksamkeit an Verbindungen mit leichter homolytischer Spaltbarkeit der Methylenprotonenbindung in 10-Position des Anthrongrundkörpers gebunden ist. Dadurch wird verständlich, daß aus der Vielzahl neuer Derivate mit Substitution in der 10-Position nur die monosubstituierten Anthronderivate eine zum Teil höher biologische Wirksamkeit aufweisen als Dithranol selbst. Ein wesentlicher Nachteil aller 10-substituierter Derivate ist: sie weisen keine wesentliche Verbesserung der Penetrationseigenschaften in die psoriatisch erkrankten hydrophileren Hautpartien auf als der reine Wirkstoff Dithranol.

Ausgehend hiervon ist es die Aufgabe der vorliegenden Erfindung, eine neue pharmazeutische Zubereitung in einer zur äußeren Anwendung geeigneten Aufmachungsform vorzuschlagen, die Derivate von Dithranol in einer Form enthält, die eine deutlich verbesserte Penetration in die psoriatisch erkrankten hydrophileren Hautpartien erlaubt als dies bisher möglich war.

Die Aufgabe wird durch die kennzeichnenden Merkmale des Patentanspruches 1 gelöst. Die Unteransprüche zeigen vorteilhafte Weiterbildungen auf. Die Verwendung der erfindungsgemäßen Zubereitung ist in Patentanspruch 7 gekennzeichnet.

Erfindungsgemäß wird somit eine pharmazeutische Zubereitung vorgeschlagen, die als Wirkstoff Phosphorsäureester von Dithranol enthält. Die in der erfindungsgemäßen pharmazeutischen Zubereitung enthaltenen Phosphorsäureester des Dithranols sind durch die nachfolgenden allgemeinen Formeln I bis IV definiert, 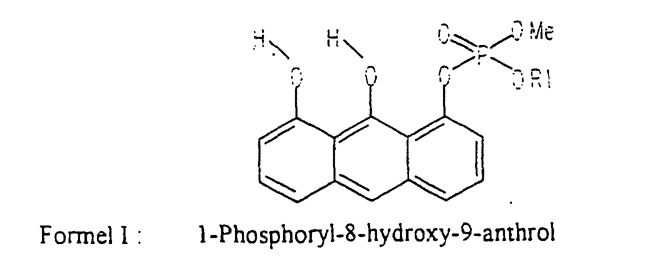 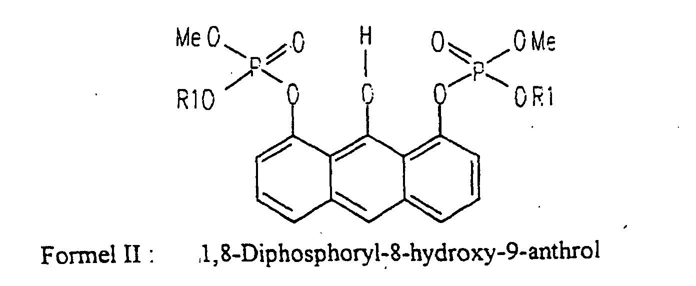 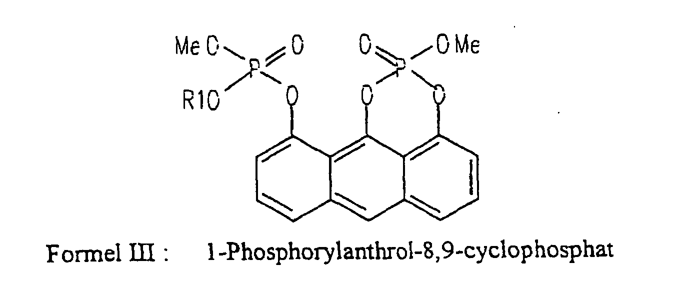 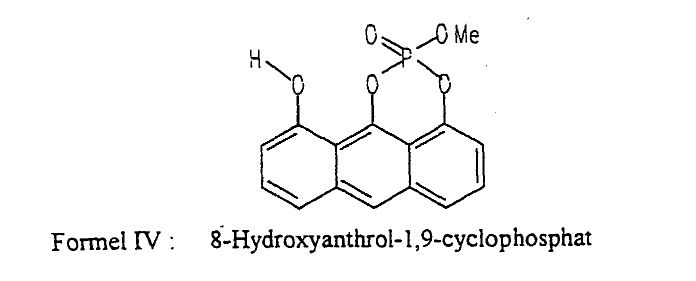 worin R¹ ausgewählt ist aus H, Arylgruppen, z. B. Phenyl und substituierte Homologe, Alkylgruppen, z.B. Methyl, Ethyl einschließlich höherer Homologe wie Dodecyl und Me, insbesondere ein Alkalimetall oder Erdalkalimetall ist. Bevorzugt ist es hierbei, wenn der Rest R¹ gleich Wasserstoff und als Alkalimetall Kalium vorhanden ist. In zahlreichen Versuchen hat es sich als besonders vorteilhaft erwiesen, wenn der Wirkstoff durch die allgemeine Formel IV definiert ist, wobei hier wiederum das Metall Kalium und R¹ gleich Wasserstoff ist.

Besonders hervorzuheben bei den erfindungsgemäßen pharmazeutischen Zubereitungen ist, daß die physikochemischen Eigenschaften der enthaltenen Wirkstoffe sich über die Art ihrer kationischen Komponenten und die Anzahl der Phosphatreste am Dithranol variieren lassen. In Form einfacher Metallsalze mit ein oder mehrwertigen Metallkationen sind diese leicht bis mäßig gut wasserlöslich. Auf diese Weise ist eine gezielte steuerbare Veränderung durch die Gegenionenart des Phosphatrestes möglich ohne die Phosphatstruktur als solches variieren zu müssen. Dabei ist die Hydrophilie dieser Verbindungen der Anzahl der Phosphatreste und damit der Anzahl der Gegenionen proportional. Die Veränderung des Hydrophilie-/Lipophilieverhaltens ist auch direkt über die Phosphatstruktur möglich. Als Monoester oder als Diphosphorsäureester an einem Anthrolrest wird die stärkste Hydratation erreicht. Es sind aber auch zyklische Anthrolphosphatstrukturen möglich (allgemeine Formeln III und IV) die in 9-Position zyklisch und ggf. in 8-Position mit einem weiteren Phosphorylrest verestert sind. Besonders hervorzuheben ist, daß die durch die Struktur- und Kombinationsvielfalt dieser Phosphorsäureester es möglich wird, Prodrug-Formen anzubieten, in denen der Wirkstoff sowohl in wäßrigen als auch in wasserfreien Zubereitungen homogen verteilt, micellar gelöst oder suspendiert vorliegt. Bevorzugt enthält die pharmazeutische Zubereitung 0,1 bis 20 Gew.-% des Wirkstoffes, besonders bevorzugt 0,5 bis 5 Gew.-% des Wirkstoffes. Die Herstellung einer derartigen pharmazeutischen Zubereitung in der der Wirkstoff homogen verteilt, micellar gelöst oder suspendiert in wäßriger oder wasserfreier Formulierung vorliegt, ist grundsätzlich aus dem Stand der Technik bekannt. Hierzu wird auf H. D. Dörfler "Grenzflächen- und Kolloidchemie", VCH Verlagsgesellschaft, Weinheim 1994, verwiesen.

Mit der neuen pharmazeutischen Zubereitung, die Prodrugs auf Phosphorsäureesterbasis enthält, ergeben sich grundsätzlich neue Anwendungsformen für die Behandlung der Psoriasis. Mit den erfindungsgemäßen Prodrugs kann der Wirkstoff aus galenisch akzeptablen Formulierungen gezielt in die psoriatisch erkrankten Hautpartien penetrieren, wobei er im Bereich der Epidermis/Dermis vollständig freigesetzt wird. Da jedoch keine merkliche Penetration der Prodrugs aus wäßrigen Formulierungen in nicht erkrankte Hautpartien festgestellt und damit deren Reizung verhindert werden kann, eignen sich diese vorzüglich zur Behandlung der Psoriasis. Mit den erfindungsgemäßen Prodrugs wird somit ein neuer Weg in der Behandlung der Psoriasis ermöglicht, indem einer der effektivsten Wirkstoffe in der Therapie mittels Waschlösungen, Lotionen oder gelartigen Formulierung verfügbar gemacht wird. Entscheidend dabei ist, daß diese Anwendungen weitestgehend ohne mechanische Beanspruchung der geschädigten und damit reizunverträglichen Haut erfolgt. Durch Bade-, Betupfungsoder sonstige Befeuchtungsroutinen bzw. sanftes Auftrages von Gelen ist es möglich, einen entsprechenden Konzentrationsunterschied an Prodrug von der wäßrigen Phase zur Cutis zu erzeugen, wodurch die Penetration von Wirkstoff in die Haut in gewünschter Weise ausgelöst wird. So läßt sich über die dem Zweck entsprechende Auswahl des Prodrugs, seine Konzentration in der Formulierung selbst, als auch über die Zeitdauer der Kontaktierung mit diesen Zubereitungen steuern. Entsprechende Zusätze wie Harnstoff fördern die schnelle Keratinolyse, begünstigen die Hydratation der Hornschicht bei starken Schuppungen oder Läsionen bzw. die Strukturierung innerhalb der Epidermis zur Verbesserung der Penetration in das Corium. Diese Faktoren lassen sich individuell auf Art und Stärke der psoriatischen Schädigung bei den jeweiligen Patienten einstellen und gestatten damit eine optimale Anpassung des Behandlungsregimes.

Zur Anwendung kommen für die Ganzkörpertherapie u.a. Badezusätze oder zur lokalen Therapie Waschlösungen, Sole zum Besprühen erkrankter Hautpartien bzw. Basislösungen zum Tränken von Hautauflagen sowie Gele. Die Kombination mit UV A-/UV B-Phototherapie bietet sich wie bisher als Behandlungsroutine ebenso an, wie eine Ergänzung zu anderen systemischen Therapien, mit dem Ziel der schnellen und wirkungsvollen Behandlung der Läsionen. Von entscheidendem Vorteil für die Anwendung von Phosphorsäureester des Dithranol als Prodrug ist die strukturbestimmte Variabilität ihrer Löslichkeit. Damit ergeben sich viele neue Möglichkeiten die Formulierungen besser auf die Erfordernisse unterschiedlicher Krankheitsbilder bei den Patienten abzustimmen.

Die Erfindung wird nachfolgend anhand von mehreren Herstellungsbeispielen für die Wirkstoffe und Formulierungsbeispielen erläutert.

### Ausführungsbeispiel 1

### Herstellung von 1-Phosphoryl-8,9-dihydroxyanthrol (Kaliumsalz)

Man rührt bei Raumtemperatur und unter Argonatmosphäre in eine Mischung von 0,1 mol Dithranol in 300 ml wasserfreiem Toluen 0,1 mol Kaliummethylat. Dabei färbt sich die Lösung von gelb nach weinrot. Unter Anlegen eines schwachen Vakuums wird zunächst das bei der Kaliumdithranolatbildung freiwerdende Methanol unter Rühren destillativ entfernt. Anschließend wird das Toluen bis zur Trockne abdestilliert.

Der Reaktionsrückstand wird mit ca. 300 ml Dioxan versetzt und unter Rühren im Eisbad auf 10 °C heruntergekühlt. Zu dieser Reaktionslösung werden 0,1 mol Phosphoroxidchlorid in 100 ml des gleichen Lösungsmittels langsam so zugetropft, daß die Innentemperatur nicht über 15 °C ansteigt. Man rührt noch eine Stunde nach, entfernt die Außenkühlung und rührt weitere zwei Stunden bei Raumtemperatur nach. Anschließend wird das Kaliumchlorid über eine Rohrfritte inert abfiltriert und mit wenig Dioxan gespült. Das Filtrat wird unter äußerer Eiskühlung mit 0,4 mol K₂CO₃ in ca. 500 ml destilliertem Wasser langsam hydrolysiert. Die Innentemperatur darf dabei nicht über 25 °C ansteigen. Der Rückstand wird unter Vakuum bis zur Trockne eingedampft, das Rohprodukt mit trockenem Dioxan aufgenommen und unter Argonatmosphäre umkristallisiert. Durch fraktionierte Kristallisation trennt man die Kaliumsalze von 1-Phosphoryl-8-hydroxyanthrol (Hauptprodukt, s. Formel IV) und 8-Hydroxyanthrol-1,9-cyclophosphat. Die Produkte werden anschließend im Vakuum über P₄O₁₀ getrocknet. Die Salze sind leicht gelbgrün gefärbt. Sie schmelzen unter Zersetzung oberhalb von 200 °C.
- ³¹P-NMR-Spektrum in Dioxan/Wasser:: -3,5 ppm (Singulett)
- Kapillarelektrophorese bei pH 8:: 1 Produktsignal hoher effektiver Leitfähigkeit
- UV/VIS (in Wasser):: typisches Anthracen-Spektrum Scharfe Absorption bei ca. 257 nm und
Absorption mit benzoider alpha-Bandenstruktur mit Max. bei ca. 372 nm

| Elementaranalysen*: C₁₄H₁₀O₆PK (M=344,31 g/mol) | | | | |
|---|---|---|---|---|
| | C: | H: | O: | P: |
| Berechnet [%] | 48,84 | 2,93 | 27,88 | 9,00 |
| Gefunden [%] | 49,17 | 3,08 | 28,04 | 8,85 |

| | | | | |
|---|---|---|---|---|
| * (sehr schwer verbrennbar) | | | | |

### Ausführungsbeispiel 2

### Herstellung von 1-Dodecylphosphoryl-8-hydroxy-9-anthron (Kaliumsalz)

In einem 500 ml Dreihalskolben werden 0,05 mol Dithranol in ca. 300 ml trockenem Dioxan vorgelegt. Unter inerten Bedingungen und Rühren werden 0,05 mol Kaliummethylat zugesetzt, wobei sich die Farbe der Reaktionslösung von gelborange über violett nach dunkelbraun verändert. Durch Anlegen eines leichten Vakuums von 150 Torr und geringem Erwärmen auf ca. 50 °C wird das entstehende Methanol aus dem Reaktionsgemisch während 2 h abgezogen. Anschließend wird zu dieser Lösung bei Raumtemperatur das Dodecyldichlorphosphat (0,05 mol) in 50 ml trockenem Dioxan inert zugesetzt. Unter Rühren und langsamen Erwärmen des Reaktionsansatzes auf 60 °C wird die Umsetzung über 3 h vervollständigt, wobei sich die Farbe der Reaktionslösung von dunkelbraun nach dunkelgelb ändert. Das voluminös anfallende Kaliumchlorid wird über ein Rohrfritte inert abfiltriert und mit wenig trockenem Dioxan gewaschen. Das Dioxan der vereinigten Filtrate wird am Rotationsverdampfer bis zur Trockne abdestilliert und anschließend mit wenig Pentan aufgenommen, um Spuren von Kaliumchlorid zu entfernen. Nach Filtration und Abdestillieren des Pentans bleibt ein braunes zähflüssiges Produkt zurück, welches mit 0,1 mol Kaliumhydrogenkarbonat, gelöst in einem Lösungsmittelgemisch aus je 100 ml destilliertem Wasser und Methanol, hydrolysiert wird. Das entstehende Kaliumsalz des 1-Dodecyl-phosphoryl-8-hydroxy-9-anthrol wird über präparative HPLC an RP-8-Phasen in einem Lösungsmittelgemisch aus Acetonitril und destilliertem Wasser gereinigt. Die Produktphase wird destillativ aufgearbeitet und schonend getrocknet. Zurück bleibt ein gelblichgrünes, zählflüssiges Öl, welches nicht zur Kristallisation neigt. Die Signale des ¹H-NMR-Spektrums (Aceton-d6) entsprechen den Erwartungswerten für den Dodecylrest bzw. des Anthracenringsystems
- ³¹P-NMR-Spektrum (Methanol-d4):: -7,45 ppm (Singulett)
- UV/VIS (in Methanol):: typisches Anthracen-Spektrum Scharfe Absorption bei 260 nm und Absorption mit benzoider alpha-Bandenstruktur bei 400 nm

| | | | |
|---|---|---|---|
| Elementaranalysen*: C₂₆H₃₄O₆PK (M=512,62 g/mol) | | | |
| | C: | H: | P: |
| Berechnet [%] | 60,91 | 6,69 | 6,04 |
| Gefunden [%] | 61,06 | 6, 58 | 5,85 |

| | | | |
|---|---|---|---|
| * (sehr schwer verbrennbar) | | | |

### Ausführungsbeispiel 3

### Herstellung von 1-Diphenylphosphoryl-8-hydroxy-9-anthron

In einem 500 ml Dreihalskolben werden 0,05 mol Dithranol in ca. 300 ml trockenem Dioxan vorgelegt. Unter inerten Bedingungen und Rühren werden 0,05 mol Kaliummethylat zugesetzt, wobei sich die Farbe der Reaktionslösung von gelborange über violett nach dunkelbraun verändert. Durch Anlegen eines leichten Vakuum von 150 Torr und geringem Erwärmen auf ca. 50 °C wird das entstehende Methanol aus dem Reaktionsgemisch während 2 h abgezogen. Anschließend wird zu dieser Lösung bei Raumtemperatur das Diphenylchlorphosphat (0,05 mol) in 50 ml trockenem Dioxan inert zugesetzt. Unter Rühren und langsamen Erwärmen des Reaktionsansatzes auf 100 °C wird die Umsetzung über 12 h vervollständigt, wobei sich die Farbe der Reaktionslösung von braun nach grüngelb ändert. Das sehr kolloiddispers anfallende Kaliumchlorid koaguliert über zwei Tage in eine sinnvoll über Rohrfritten abfiltrierbare Form. Das Kaliumchlorid wird mit wenig Dioxan gewaschen. Die vereinigten Filtrate werden bis zur Trockne abdestilliert, zur Abtrennung von Kaliumchloridspuren abermals mit Pentan versetzt, filtriert und nochmals bis zur Trockne abdestilliert. Das verbleibende rötlich- bis orangefarbene Produkt ist von sehr zäher, öliger Konsistenz.

Durch präparative HPLC wird dieses Produkt an RP-8-Phasen mit Acetonitril und Äthanol (als Gradient) gereinigt. Das Produkt bleibt nach Abdestillieren des Lösungsmittelgemisches als orange bis rötlich gefärbtes zähes Öl übrig.

Die ¹H- und ¹³C-NMR-Spektren (Aceton-d6) sind im Bereich der Phenylestergruppen mit den Signalen der Anthracenringatome des Anthrongerüstes überlagert und können deshalb nicht eindeutig quantitativ ausgewertet werden. Eindeutig ist das ³¹P-NMR-Spektrum.
- ³¹P-NMR-Spektrum (Methanol-d4):: -17,8 ppm (Singulett)
- UV/VIS (in Methanol):: Anthracen-Spektrum mit überlagerten Benzenbanden (Absorption bei 250 - 270 nm) Absorption mit benzoider alpha-Bandenstruktur bei 400 nm

| | | | |
|---|---|---|---|
| Elementaranalysen*: C₂₆H₁₉O₆P (M⁺=458,4 g/mol) | | | |
| | C: | H: | P: |
| Berechnet [%) | 68,12 | 4,18 | 6,76 |
| Gefunden [%] | 67,97 | 4,25 | 6,63 |

| | | | |
|---|---|---|---|
| * (sehr schwer verbrennbar) | | | |

### Ausführungsbeispiel 4

### Herstellung einer amphiphilen Creme mit hydrophilem Phosphorsäureester des Dithranols

### Herstellung

Glycerolmonostearat 60, Cetylalkohol, mittelkettige Triglyceride und weißes Vaselin werden im Wasserbad auf etwa 60 °C erhitzt und anteilsweise mit der auf die gleiche Temperatur erwärmten Mischung von Makrogolglycerolmonostearat, Propylenglykol, Wasser und dem Kaliumsalz des 1-Phosphoryl-8,9-dihydroxyanthrols versetzt. Die Creme wird bis zum Erkalten ständig gerührt. Das dabei verdunstende Wasser wird ergänzt.

Diese Creme kann anschließend bei engstem Spalt durch den Dreiwalzenstuhl gegeben werden. Sie ist farblos, fast geruchlos, weich und mit Wasser von der Haut abwaschbar.

### Zusammensetzung

100 g Zubereitung enthalten:

| | |
|---|---|
| Glycerolmonostearat 60 | 4,0 g |
| Cetylalkohol | 6,0 g |
| Mittelkettige Triglyceride | 7,5 g |
| Weißes Vaselin | 25,5 g |
| Macrogolglycerolmonostearat | 7,0 g |
| Propylenglykol | 9,5 g |
| Wasser | 38,5 g |
| 1-Phosphoryl-8,9-dihydroxyanthrol | 2,0 g |
| (Kaliumsalz) | |

### Ausführungsbeispiel 5

### Herstellung einer amphiphilen Creme mit lipophilem Phosphorsäureester des Dithranols

### Herstellung

Glycerolmonostearat 60, Cetylalkohol, mittelkettige Triglyceride, weißes Vaselin und 1-Diphenylphosphoryl-8-hydroxy-9-anthron werden im Wasserbad auf etwa 60 °C erhitzt und anteilsweise mit der auf die gleiche Temperatur erwärmten Mischung von Makrogolglycerolmonostearat, Propylenglykol und Wasser versetzt. Die Creme wird bis zum Erkalten ständig gerührt. Das dabei verdunstende Wasser wird ergänzt.

Diese Creme kann anschließend bei engstem Spalt durch den Dreiwalzenstuhl gegeben werden. Sie ist farblos, fast geruchlos, weich und mit Wasser von der Haut abwaschbar.

### Zusammensetzung

100 g Zurbereitung enthalten:

| | |
|---|---|
| Glycerolmonostearat 60 | 4,0 g |
| Cetylalkohol | 6,0 g |
| Mittelkettige Triglyceride | 7,0 g |
| Weißes Vaselin | 24,0 g |
| 1-Diphenylphosphoryl-8-hydroxy-9-anthron | 2,0 g |
| Macrogolglyerolmonostearat | 7,0 g |
| Propylenglykol | 10,0 g |
| Wasser | 40,0 g |

## Patentansprüche

1. Pharmazeutische Zubereitung in einer zur äußeren Anwendung geeigneten Aufmachungsform enthaltend als Wirkstoff ein Derivat von Dithranol,
**dadurch gekennzeichnet**
**daß** des Derivat ausgeswählt ist aus Verbindungen der allgemeinen Formeln I bis IV 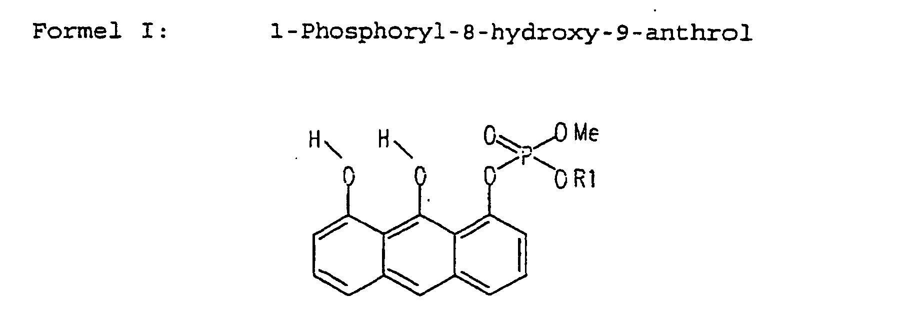 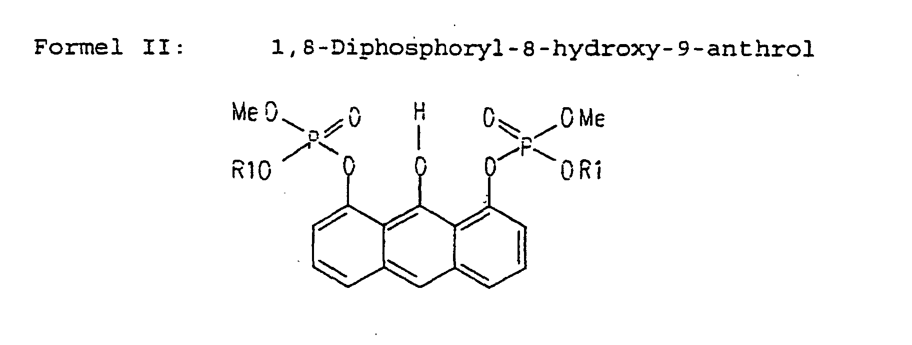 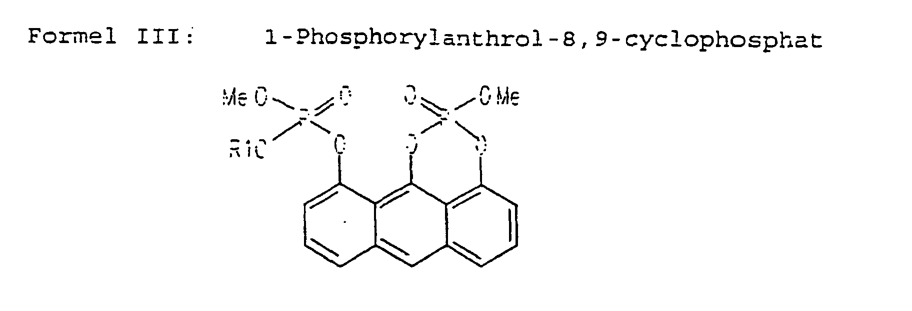 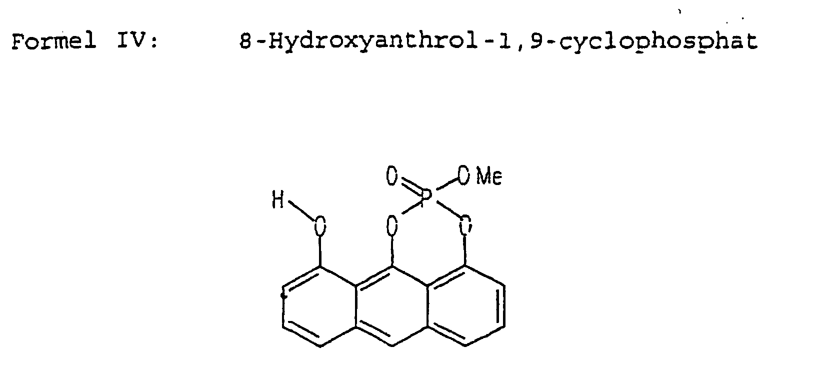 worin R¹ = H, Phenyl oder Ethyl oder Dodecyl und Me ein Alkalimetall oder Erdalkalimetall ist.

2. Pharmazeutische Zubereitung nach Anspruch 1, **dadurch gekennzeichnet, daß** R¹ = H und Me = K ist.

3. Pharmazeutische Zubereitung nach Anspruch 1, **dadurch gekennzeichnet, daß** sie einen Wirkstoff der allgemeinen Formel IV enthält, wobei Me = K und R¹ = H ist.

4. Pharmazeutische Zubereitung nach mindestens einem der Ansprüche 1 bis 3, **dadurch gekennzeichnet, daß** sie ein Gemisch von Wirkstoffen der allgemeinen Formeln I bis IV enthält.

5. Pharmazeutische Zubereitung nach mindestens einem der Ansprüche 1 bis 4, **dadurch gekennzeichnet, daß** 0,1 bis 20 Gew.-% des Wirkstoffes in der Zubereitung homogen verteilt, micellar gelöst oder suspendiert in wäßriger Formulierung vorliegt.

6. Pharmazeutische Zubereitung nach mindestens einem der Ansprüche 1 bis 4, **dadurch gekennzeichnet, daß** 0,1 bis 20 Gew.-% des Wirkstoffes homogen verteilt, micellar gelöst oder suspendiert in einer im wesentlichen wasserfreien Formulierung vorliegt.

7. Verwendung mindestens eines Dithranol-Derivates der allgemeinen Formeln I bis IV zur Herstellung einer pharmazeutischen Zubereitung zur reizarmen Therapie der Psoriasis.

## Claims

1. Pharmaceutical preparation in a presentational form which is suitable for external application, containing a derivative of dithranol as active ingredient,
**characterised in that**
the derivative is selected from compounds of the general formulae I to IV 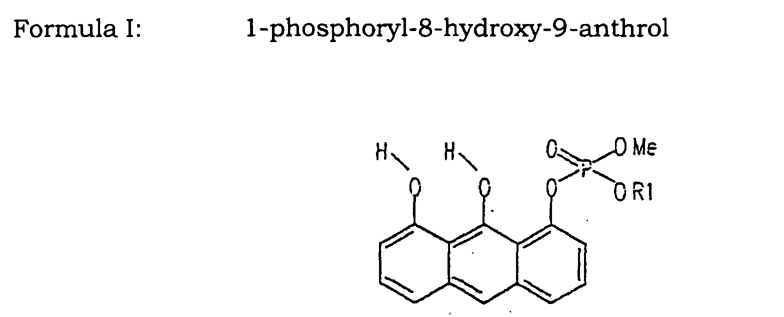 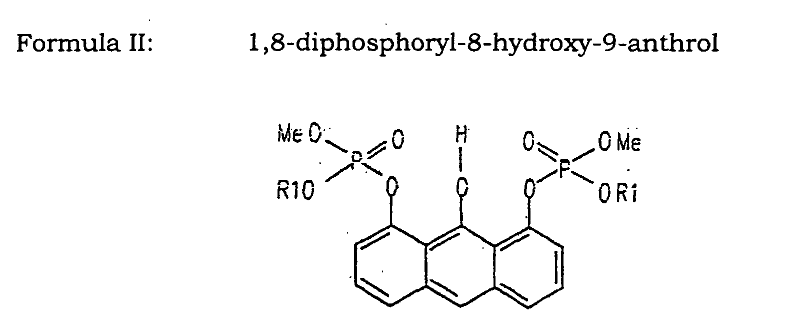 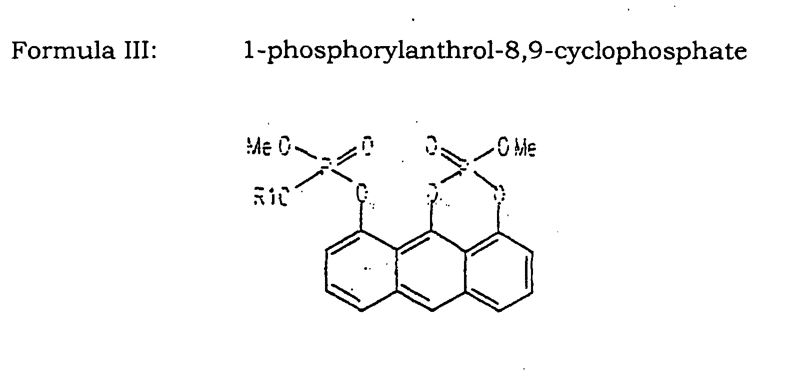 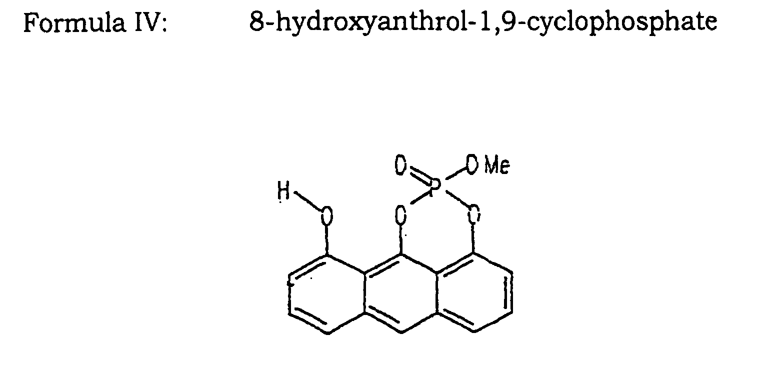 wherein R¹ = H, phenyl or ethyl or dodecyl and Me is an alkali metal or alkaline earth metal.

2. Pharmaceutical preparation according to claim 1, **characterised in that** R¹ = H and Me = K.

3. Pharmaceutical preparation according to claim 1, **characterised in that** it contains an active ingredient of the general formula IV, wherein Me = K and R¹ = H.

4. Pharmaceutical preparation according to at least one of the claims 1 to 3, **characterised in that** it contains a mixture of active ingredients of the general formulae I to IV.

5. Pharmaceutical preparation according to at least one of the claims 1 to 4, **characterised in that** 0.1 to 20% by weight of the active ingredient is present in the preparation, homogeneously distributed, dissolved in a micellar manner or suspended in an aqueous formulation.

6. Pharmaceutical preparation according to at least one of the claims 1 to 4, **characterised in that** 0.1 to 20% by weight of the active ingredient is present, homogeneously distributed, dissolved in a micellar manner or suspended in a substantially water-free formulation.

7. Use of at least one dithranol derivative of the general formulae I to IV for producing a pharmaceutical preparation for the non-irritant therapy of psoriasis.

## Revendications

1. Préparation pharmaceutique sous une forme de présentation appropriée à l'usage externe, contenant en tant que substance active un dérivé de dithranol, **caractérisée en ce que** le dérivé est choisi parmi les composés de formules générales I à IV 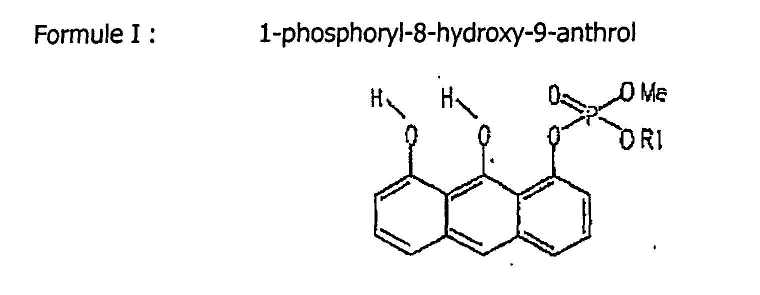 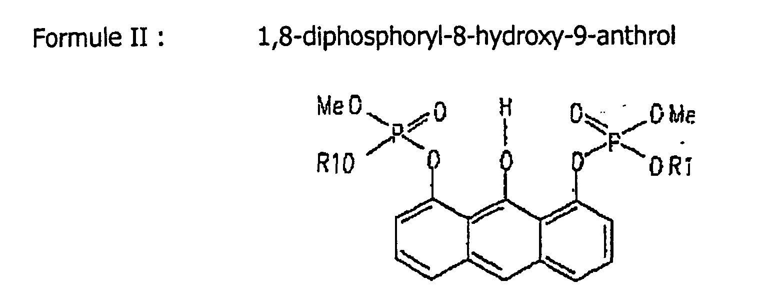 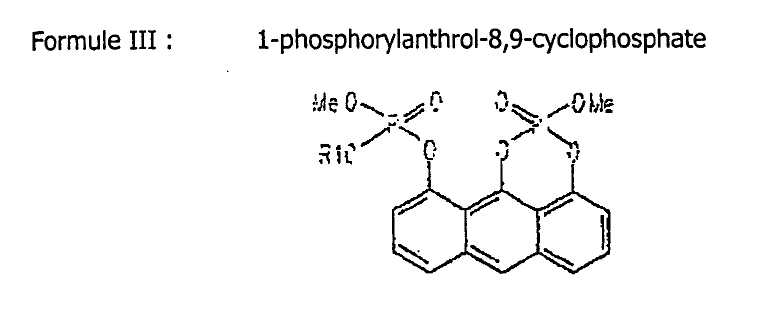 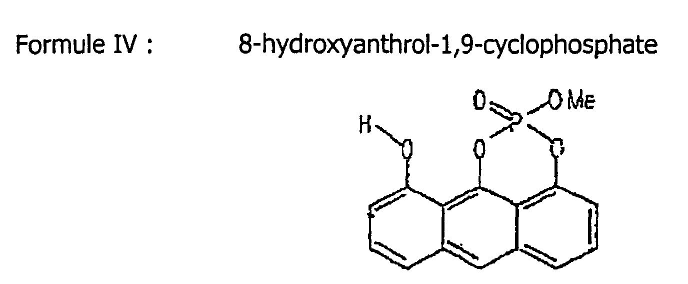 dans lesquelles
R¹ = H, phényle ou éthyle ou dodécyle et
Me est un métal alcalin ou alcalino-terreux.

2. Préparation pharmaceutique selon la revendication 1, **caractérisée en ce que** R¹ = H et Me = K.

3. Préparation pharmaceutique selon la revendication 1, **caractérisée en ce qu'**elle contient une substance active de formule générale IV, dans laquelle Me = K et R¹ = H.

4. Préparation pharmaceutique selon au moins l'une quelconque des revendications 1 à 3, **caractérisée en ce qu'**elle contient un mélange de substances actives de formules générales I à IV.

5. Préparation pharmaceutique selon au moins l'une quelconque des revendications 1 à 4, **caractérisée en ce que** de 0,1 à 20 % en poids de la substance active sont présents dans la préparation, sous forme de dispersion homogène, solution micellaire ou suspension en formulation aqueuse.

6. Préparation pharmaceutique selon au moins l'une quelconque des revendications 1 à 4, **caractérisée en ce que** de 0,1 à 20 % en poids de la substance active sont présents dans la préparation, sous forme de dispersion homogène, solution micellaire ou suspension en formulation pratiquement anhydre.

7. Utilisation d'au moins un dérivé de dithranol de formules générales I à IV, pour la production d'une préparation pharmaceutique destinée au traitement peu irritant du psoriasis.
